# FASCICULE DE BREVET EUROPEEN

(11) **EP 0 804 445 B1**
(45) Date de publication et mention de la délivrance du brevet: **01.03.2000**
(21) Numéro de dépôt: 95934197.5
(22) Date de dépôt: 11.10.1995
(51) Int. Cl.: C07F 17/00, C07F 17/02

(54) **DERIVES METALLOCENIQUES DU DIARYLETHYLENE, LEURS PROCEDES DE PREPARATION ET COMPOSITIONS PHARMACEUTIQUES CONTENANT LESDITS DERIVES**
DIARYLETHYLEN-METALLOGENDERIVATE, VERFAHREN ZU DEREN HERSTELLUNG UND DIESE ENTHALTENDE ARZNEIZUSAMMENSETZUNGEN
METALLOCENIC DIARYLETHYLENE DERIVATIVES, METHODS FOR THEIR PREPARATION AND PHARMACEUTICAL COMPOSITIONS CONTAINING SAME

(30) Priorité: 12.10.1994 FR 9412151
(43) Date de publication de la demande: 05.11.1997
(73) Titulaire: Modelisation et Mise au Point de Molecules Medicanales - "4M", 75016 Paris (FR)
(72) Inventeur: JAOUEN, Gérard, F-94240 L'Hay-les-Roses (FR); RAYNAUD, Jean-Pierre, F-75016 Paris (FR); TOP, Siden, F-91090 Lisses (FR)
(74) Mandataire: Orès, Bernard
(86) Numéro de dépôt international: FR9501323
(87) Numéro de publication internationale: WO9611935

(56) Documents cités:
- EP-A- 0 153 636
- FR-A- 2 352 825

## Description

La présente Invention se rapporte à des dérivés métallocéniques du diaryléthylène, à leurs procédés de préparation ainsi qu'à des compositions pharmaceutiques contenant lesdits dérivés.

Certaines classes de dérivés du triaryléthylène possèdent des représentants qui, tels le tamoxifène ou la nafoxidine, associent à de faibles propriétés oestrogéniques des propriétés antioestrogéniques marquées qui sont à la base de leur utilisation comme anticonceptionnels oraux (RAY et al., J. Med. Chem., 1994, 37, 696), comme inducteurs de l'ovulation et surtout comme antitumoraux (JORDAN et MURPHY, Endocr. Rev., 1990, 11, 578 ; DORE et al., J. Med. Chem, 1992, 35, 573 ; KYM et al., J. Med. Chem., 1993, 36, 3910).

Cette dualité de propriétés, oestrogénique faible et antioestrogénique prononcée, résulte sans doute de la présence de deux composantes fonctionnelles distinctes dans la structure de ces composés. Il s'agit, d'une part, d'une entité de type trans stilbène qui simule la structure de l'oestrogène synthétique, le diéthylstilbestrol et, d'autre part, d'un résidu additionnel polaire consistant, par exemple, en un groupe du type (ω-tert-aminoalcoxy)phényle qui interfère avec l'initiation de l'activité oestrogénique et se trouve responsable de l'activité antioestrogénique.

L'hypothèse de la présence de Zn⁺⁺ acide coordinant au voisinage du site d'association de l'hormone a été récemment avancée pour expliquer ce comportement (JAOUEN et al., Acc. Chem. Res., 1993, 26, 361) .

Quelle que soit la nature du processus moléculaire mis en jeu, le tamoxifène est largement utilisé comme thérapie adjuvante dans le contrôle des cancers du sein répondant positivement à l'analyse des récepteurs de l'oestradiol. Cette molécule a un effet positif sur la survie des patients et est par ailleurs bien tolérée.

Toutefois, comme l'action du tamoxifène est tumoristatique plutôt que tumoricide, son utilisation dans cette indication justifie généralement une administration prolongée au cours de laquelle certains patients développent une résistance au traitement (WOLF et JORDAN, Breast Cancer Res. Treat., 1993, 27, 27). A l'extrême, certaines tumeurs du sein et de l'endomètre finissent par se développer par stimulation au tamoxifène.

C'est la raison pour laquelle le problème se pose de développer de nouvelles molécules douées de propriétés antitumorales et propres à remplacer les antioestrogènes triaryléthyléniques de l'Art Antérieur et notamment le tamoxifène dans leurs applications tumorales comme le traitement des cancers du sein oestrogéno-dépendants.

On sait, par ailleurs, par la Demande de Brevet Européen n° 0 153 636, que des sels de métallicénium formés d'une association entre un cation métallicénium et un anion complexant ou fortement encombrant de type tétrachloro- ou tétrabromoferrate, tétrafluoro- ou tétraphénylborate, hexafluorophosphate, quinonure ou encore polyhalogénure, présenteraient une activité cytostatique non spécifique leur conférant une utilité dans le traitement des pathologies cancéreuses en général.

Le problème est résolu par la présente Invention qui propose une nouvelle famille de composés susceptibles de servir avantageusement de substituts aux antioestrogènes triaryléthyléniques de l'Art Antérieur, notamment au tamoxifène, en raison de ce qu'ils présentent une activité antitumorale supérieure à celle de ce dernier, des procédés de préparation de ces nouveaux composés ainsi que des compositions pharmaceutiques contenant lesdits composés.

La présente Invention a, donc, pour objet des composés de formule générale (I) : 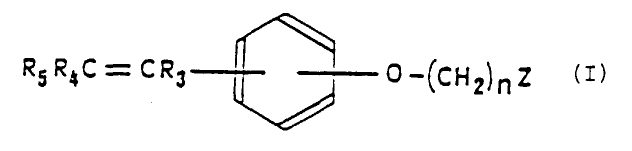 dans laquelle :
le groupe -O(CH₂)ₙZ se trouve en position méta ou para par rapport au groupe -CR₃=CR₄R₅,
n représente un nombre entier compris entre 2 et 10,
Z représente un coordinat basique ou polaire choisi parmi les groupes -NR₁R₂ dans lesquels R₁ et R₂, identiques ou différents, représentent un groupe alkyle en C₁ à C₆ éventuellement substitué par un ou plusieurs atomes d'halogène, les groupes hétérocycliques renfermant un atome d'azote ou les groupes -OR₁, -SR₁, -SOR₁ ou -SO₂R₁ dans lesquels R₁ a la même signification que précédemment,
et dans laquelle :
   * soit R₃ représente un groupe phényle éventuellement substitué par un ou plusieurs atomes d'hydrogène ou atomes d'halogène ou groupes hydroxyle ou groupes alkyle ou alcoxy en C₁ à C₃ et R₅ représente un groupe métallocène, tandis que R₄ représente un atome d'hydrogène ou un atome d'halogène ou un groupe NO₂ ou un groupe chloroéthyle ou un groupe CN ou encore un groupe alkyle en C₁ à C₃ dont le carbone terminal peut éventuellement être lié au carbone situé en position 2 du groupe phényle de R₃,
   * soit R₃ représente un groupe métallocène et R₅ représente un groupe phényle éventuellement substitué par un ou plusieurs atomes d'hydrogène ou atomes d'halogène ou groupes hydroxyle ou groupes alkyle ou alcoxy en C₁ à C₃, tandis que R₄ représente un atome d'hydrogène ou un atome d'halogène ou un groupe NO₂ ou un groupe chloroéthyle ou un groupe CN ou encore un groupe alkyle en C₁ à C₃,
   sous toutes leurs formes stéréoisomères ainsi que leurs sels.

De manière inattendue, les Inventeurs ont, en effet, découvert que la fixation sur un même squelette d'une composante antioestrogénique et d'une entité métallocénique conduit à des composés doués de propriétés tumoristatiques et tumoricides marquées, supérieures à celles des antioestrogènes de l'Art Antérieur, et de ce fait, propres à être utilisés comme antitumoraux, notamment dans le traitement des cancers du sein oestrogéno-dépendants.

Dans ce qui précède et ce qui suit, l'expression "alkyle en C₁ à C₆" désigne tout groupe alkyle n'ayant pas plus de 6 atomes de carbone, linéaire ou ramifié, tel que par exemple les radicaux méthyle, éthyle, propyle, butyle, isopropyle ou encore isobutyle.

De façon similaire, l'expression "alkyle en C₁ à C₃" désigne tout groupe alkyle n'ayant pas plus de 3 atomes de carbone, linéaire ou ramifié.

Par groupe "alcoxy en C₁ à C₃", la présente Demande vise à désigner tout groupe alcoxy n'ayant pas plus de 3 atomes de carbone comme par exemple un groupe méthoxy, éthoxy, propoxy ou isopropoxy.

L'expression "groupe hétérocyclique renfermant un atome d'azote" désigne un hétérocycle azoté ne comprenant pas plus de 6 atomes dans le cycle comme par exemple un groupe N-pipéridino, N-morpholino ou N-pyrrolidino.

L'expression "halogène" désigne le chlore, le fluor, le brome ou l'iode.

Dans une forme de réalisation préférée de l'Invention, le groupe métallocène répond à la formule générale (II) : 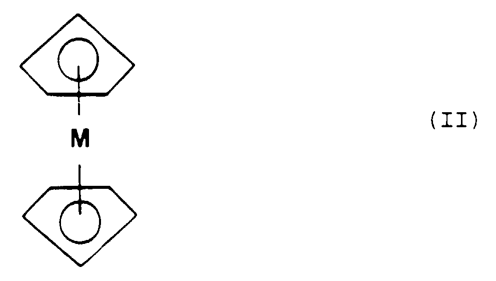 dans laquelle M représente un atome métallique du groupe VIII de la Classification Périodique des éléments chimiques, ledit atome pouvant être éventuellement sous une forme oxydée.

Dans une autre forme de réalisation de l'Invention, le groupe métallocène répond à la formule générale (III) : 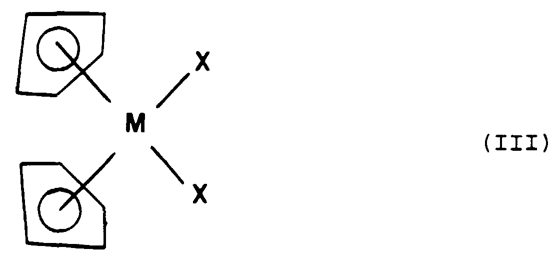 dans laquelle :
M représente un atome métallique choisi parmi le titane, le vanadium, le nobium, le hafnium ou le molybdène, et
X représente un atome d'halogène choisi parmi le chlore, le brome ou l'iode.

L'Invention a plus particulièrement pour objet les composés tels que définis ci-dessus, caractérisés en ce qu'ils répondent à la formule particulière (I-a) : 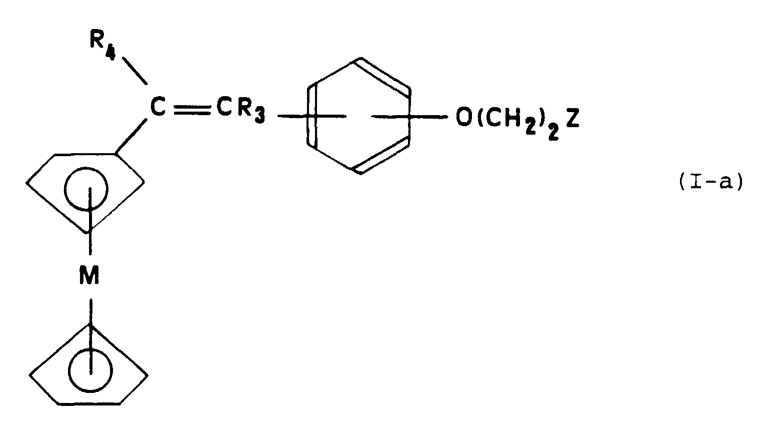 dans laquelle :
le groupe -O(CH₂)₂Z se trouve en position para par rapport au groupe -CR₃=CR₄R₅,
Z représente un groupe -NR₁R₂ dans lequel R₁ et R₂, identiques ou différents, représentent un groupe méthyle ou un groupe éthyle, ou bien Z représente un groupe N-pyrrolidino,
R₃ représente un groupe phényle éventuellement substitué par un groupe hydroxyle ou par un groupe méthoxy,
R₄ représente un atome d'hydrogène ou un atome d'halogène ou un groupe NO₂ ou un groupe chloroéthyle ou un groupe CN ou encore un groupe éthyle, et
M représente un atome métallique choisi parmi le fer, le ruthénium ou l'osmium, éventuellement sous sa forme oxydée.

Ces composés sont isomérisables de sorte que dans ce qui suit, on désignera par isomères "trans" les composés de formule (I-a) présentant la configuration ci-après : 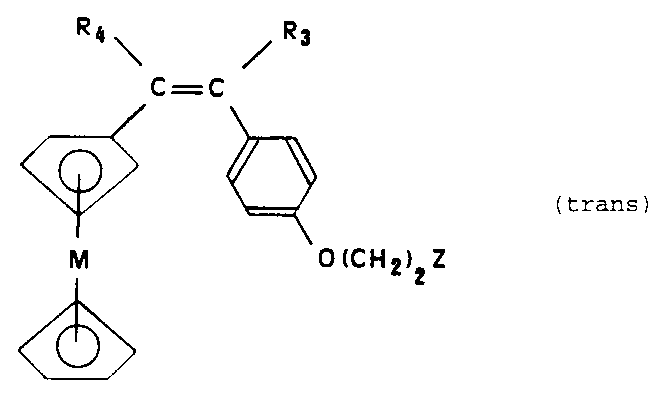 dans laquelle le groupe R₃ et le groupe métallocène sont en position "trans" l'un par rapport à l'autre de part et d'autre de la liaison oléfinique,
tandis qu'on désignera par isomères "cis" les composés métallocéniques présentant la configuration ci-après : 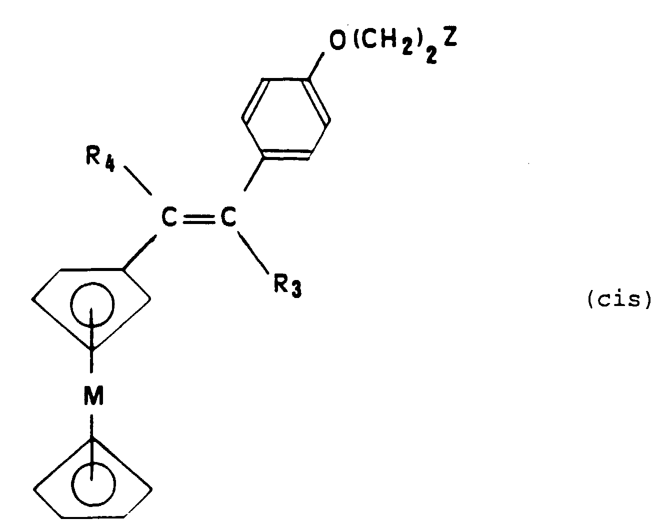 dans laquelle le groupe R₃ et le groupe métallocène sont en position "cis" l'un par rapport à l'autre de part et d'autre de la liaison oléfinique.

Parmi les composés de formule (I-a), on préfère notamment ceux répondant à la formule particulière (I-al) dans laquelle R₁ et R₂ représentent un groupe méthyle, R₃ représente un groupe phényle substitué en para par un groupe hydroxyle et R₄ représente un groupe éthyle.

La formule particulière (I-al) est représentée ci-après dans la configuration "trans" :

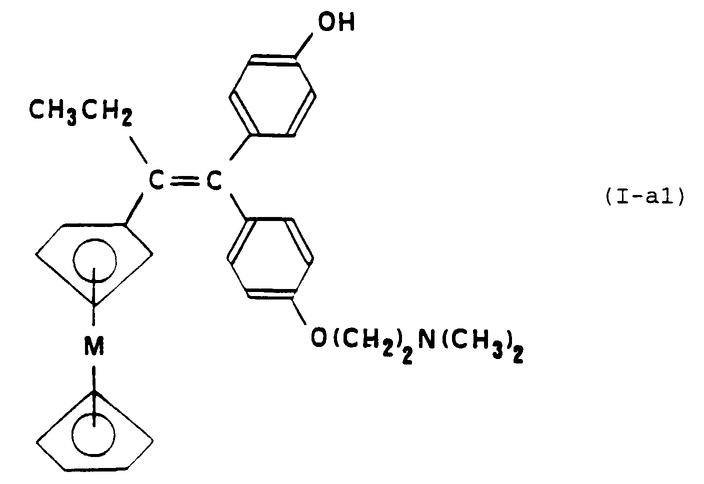

Préférentiellement, M représente un atome de fer et le composé métallocénique correspondant est le 1-[4-(2-diméthylaminoéthoxy) phényl]-1-(4-hydroxyphényl)-2-ferrocényl-1-butène.

De manière particulièrement préférée, le composé métallocénique est l'isomère "trans" du 1-[4-(2-diméthylaminoéthoxy) phényl]-1-(4-hydroxyphényl)-2-ferrocényl-1-butène.

Parmi les composés de formule (I-a), on préfère également les composés répondant à la formule particulière (I-a2) dans laquelle R₁ et R₂ représentent un groupe méthyle, R₃ représente un groupe phényle et R₄ représente un groupe éthyle.

La formule particulière (I-a2) est représentée ci-après dans la configuration "trans" :

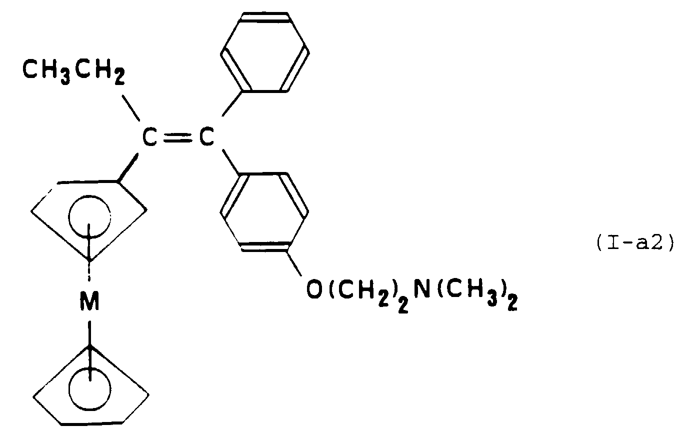

De préférence, M représente un atome de fer et le composé métallocénique correspondant est le 1-[4-(2-diméthylaminoéthoxy) phényl]-1-phényl-2-ferrocényl-1-butène.

L'Invention a également plus particulièrement pour objet les composés répondant à la formule générale (I), caractérisés en ce que, dans cette formule (I) :
le groupe -O(CH₂)ₙZ se trouve en position para par rapport au groupe -CR₃=CR₄R₅,
n est égal à 2 et Z représente un groupe N-pyrrolidino,
R₃ représente un groupe phényle substitué en para par un groupe hydroxyle ou alcoxy en C₁ à C₃, tandis que R₄ représente un groupe alkyle en C₂ dont le carbone terminal est lié au carbone situé en position 2 du groupe phényle de R₃,
M représente un atome métallique choisi parmi le fer, le ruthénium ou l'osmium, éventuellement sous sa forme oxydée.

Ces composés répondent à la formule particulière (I-b) : 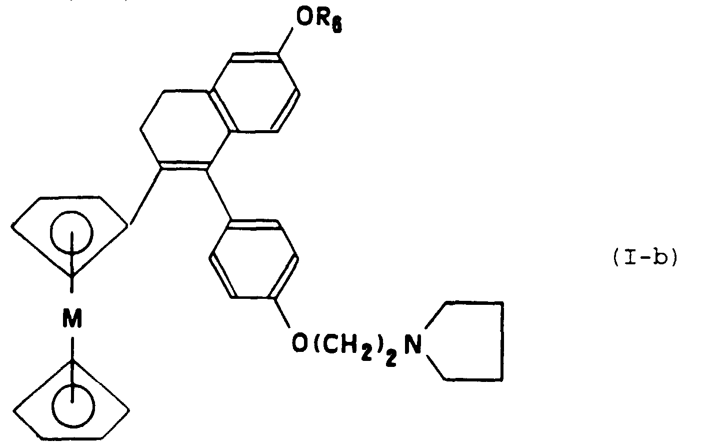 dans laquelle R₆ représente un atome d'hydrogène ou un groupe alkyle en C₁ à C₃.

Conformément à l'Invention, les composés de formule générale (I) peuvent être préparés selon un procédé caractérisé en ce que :
a) on fait réagir un composé de formule générale (IV) : 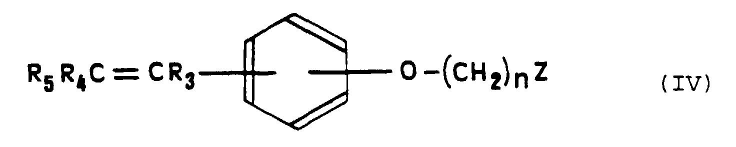 dans laquelle :
   le groupe -O(CH₂)ₙZ est en position méta ou para par rapport au groupe -CR₃=CR₄R₅,
   n représente un nombre entier compris entre 2 et 10,
   Z représente un coordinat basique ou polaire choisi parmi les groupes -NR₁R₂ dans lesquels R₁ et R₂, identiques ou différents, représentent un groupe alkyle en C₁ à C₆ éventuellement substitué par un ou plusieurs atomes d'halogène, les groupes hétérocycliques renfermant un atome d'azote ou les groupes -OR₁, -SR₁, -SOR₁ ou -SO₂R₁ dans lesquels R₁ a la même signification que précédemment,
   et dans laquelle :
      * soit R₃ représente un groupe phényle éventuellement substitué par un ou plusieurs atomes d'hydrogène ou atomes d'halogène ou groupes hydroxyle ou groupes alkyle ou alcoxy en C₁ à C₃ et R₅ représente un atome de brome, tandis que R₄ représente un atome d'hydrogène ou un atome d'halogène ou un groupe NO₂ ou un groupe chloroéthyle ou un groupe CN ou encore un groupe alkyle en C₁ à C₃ dont le carbone terminal peut éventuellement être lié au carbone situé en position 2 du groupe phényle de R₃,
      * soit R₃ représente un atome de brome et R₅ représente un groupe phényle éventuellement substitué par un ou plusieurs atomes d'hydrogène ou atomes d'halogène ou groupes hydroxyle ou groupes alkyle ou alcoxy en C₁ à C₃, tandis que R₄ représente un atome d'hydrogène ou un atome d'halogène ou un groupe NO₂ ou un groupe chloroéthyle ou un groupe CN ou encore un groupe alkyle en C₁ à C₃,
      avec un sel métallique de cyclopentadiènyle pour obtenir la substitution de l'atome de brome par un groupe cyclopentadiène ;
b) on fait réagir le composé obtenu à l'étape a) avec une base forte pour obtenir la formation d'un anion cyclopentadiènyle,
c) on fait réagir le composé obtenu à l'étape b) avec un composé organométallique de formule :
   Cp-M-(CO)₂-X dans laquelle Cp représente un groupe cyclopentadiène, M représente un atome métallique du groupe VIII de la Classification Périodique des éléments chimiques et X représente un atome d'halogène choisi parmi le chlore, le brome ou l'iode,
   ou avec un composé organométallique de formule : Cp-M-Xₘ dans laquelle Cp et X ont la même signification que précédemment, M représente un atome métallique choisi parmi le titane, le vanadium, le nobium, le hafnium ou le molybdène et m est un nombre entier égal à 3 ou 4.

Les composés de formule générale (IV) peuvent être préparés selon les procédés qui ont été proposés dans l'Art Antérieur pour la synthèse des antioestrogènes triaryléthyléniques (Cur. Med. Chem., 1994, 1, 61-104).

Un sel métallique de cyclopentadiènyle convenable pour la mise en oeuvre de l'étape a) de ce procédé est représenté par le cyclopentadiènate de sodium.

La base forte utile pour la mise en oeuvre de l'étape b) peut par exemple être NaH ou NaNH₂.

Bien que les composés de formule particulière (I-al) puissent être préparés selon le procédé de préparation décrit ci-dessus, l'Invention a également pour objet un procédé de préparation spécifique des composés de formule (I-a1), caractérisé en ce que :
a) on soumet un ester métallocénique de formule (V) : 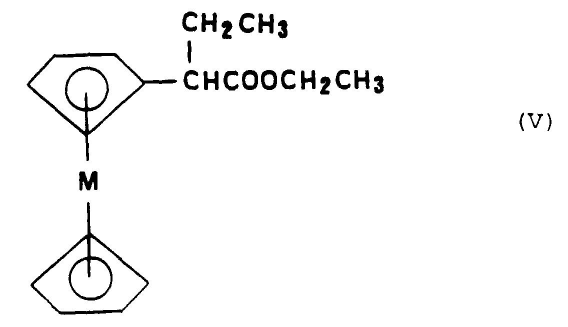 dans laquelle M représente un atome métallique choisi parmi le fer, le ruthénium ou l'osmium, à une réaction d'addition avec l'organolithien CH₃-O-C₆H₄-Li ou un organomagnésien de formule : CH₃-O-C₆H₄-MgX dans laquelle X représente un atome de chlore, de brome ou d'iode, qui conduit à la formation d'un alcool intermédiaire, suivie d'une réaction de deshydratation dudit alcool,
b) on soumet le composé obtenu à l'étape a) à une réaction de déméthylation,
c) on soumet le composé obtenu à l'étape b) à une réaction d'alkylation par l'éthanolate de sodium et l'hydrochlorure de chlorure de 2-diméthylaminoéthyle.

On obtient ainsi un mélange des deux isomères "cis" et "trans" d'un composé de formule (I-a1).

La deshydratation de l'alcool intermédiaire peut avantageusement être réalisée par l'action d'un acide fort tel que l'acide chlorhydrique.

La réaction de déméthylation peut avantageusement être réalisée par l'action du tribromure de bore en présence d'un solvant approprié tel que par exemple le dichlorométhane.

Dans un mode de mise en oeuvre préféré du procédé conforme à l'Invention, ce procédé comprend préalablement la préparation de l'ester métallocénique de formule (V) par estérification de l'acide métallocénylacétique correspondant, suivie d'une réaction d'alkylation du carbone en position α du cycle métallocényle dudit ester.

L'estérification de l'acide métallocénylacétique peut être réalisée au moyen d'une base forte telle que le tertiobutylate de potassium (t-BuOK).

Un réactif d'alkylation convenable est représenté par l'iodoéthane. Cette alkylation est avantageusement réalisée en présence d'une base forte telle que par exemple le t-BuOK ou l'hexaméthyldisilazane de sodium ou de potassium et d'un solvant approprié comme par exemple le diméthylsulfoxyde (DMSO).

L'acide métallocénylacétique peut être préparé selon la méthode décrite dans la littérature (LEDNICER et al., J. Org. Chem., 1958, 23, 653) qui consiste à faire réagir un iodure de métallocénylméthyl-triméthyl ammonium avec du cyanure de potassium pour obtenir le métallocénylacétonitrile correspondant puis à hydrolyser ce dernier.

Dans la mesure où la préparation d'un dérivé métallocénique de formule (I-a1) selon ce procédé conduit toujours à l'obtention des deux isomères "cis" et "trans" dudit dérivé et où généralement seul l'un des deux isomères, à savoir l'isomère "trans", présente des propriétés antioestrogéniques prononcées, il peut être avantageux de procéder à une séparation des deux isomères obtenus à l'issue de l'étape c) précitée.

Conformément à l'Invention, le procédé de préparation spécifique des composés de formule (I-a1) comprend de plus une étape de séparation des isomères "cis" et "trans" de ces composés.

Cette séparation peut avantageusement être réalisée par cristallisation fractionnée. Il est, toutefois, possible d'utiliser d'autres techniques de l'Art Antérieur telles que par exemple une chromatographie sur couche mince à l'aide d'un éluant approprié.

Bien que les composés de formule particulière (I-a2) puissent également être obtenus par le procédé de préparation des composés de formule générale (I), l'Invention a aussi pour objet un procédé de préparation spécifique de ces composés de formule particulière (I-a2) qui est caractérisé en ce que :
a) on fait réagir un chlorure d'acide métallocénique de formule (VI) : 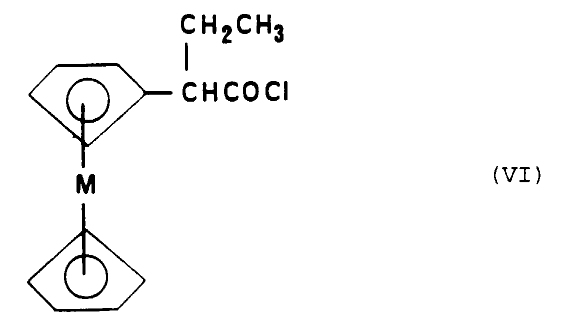 dans laquelle M représente un atome métallique choisi parmi le fer, le ruthénium ou l'osmium, avec du diphényle de zinc pour obtenir la substitution de l'atome de chlore par un groupe phényle,
b) on soumet le composé obtenu à l'étape a) à une réaction d'addition avec l'organolithien (CH₃)₂-N-(CH₂)₂-O-C₆H₄-Li ou un organomagnésien de formule : (CH₃)₂-N-(CH₂)₂-O-C₆H₄-MgX dans laquelle X représente un atome d'halogène choisi parmi le chlore, le brome ou l'iode, qui conduit à la formation d'un alcool intermédiaire, suivie d'une réaction de deshydratation dudit alcool.

On obtient ainsi un mélange des deux isomères "cis" et "trans" d'un composé de formule (I-a2).

Le chlorure d'acide métallocénique peut être obtenu par l'action du trichlorure ou du pentachlorure de phosphore ou du chlorure de thionyle ou encore du triphosgène sur l'acide métallocényl-2-butanoïque correspondant.

La deshydratation de l'alcool intermédiaire peut être réalisée par l'action d'un acide fort tel que l'acide chlorhydrique.

La préparation d'un composé de formule (I-a2) selon ce procédé conduit toujours à l'obtention des deux isomères "cis" et "trans" dudit composé de sorte qu'il comprend en outre une étape de séparation de ces isomères "cis" et "trans", laquelle peut être réalisée par exemple par cristallisation fractionnée.

La présente Invention a, aussi, pour objet un procédé de préparation particulier des composés de formule (I-b), caractérisé en ce que :
a) on fait réagir une tétralone de formule (VII) : 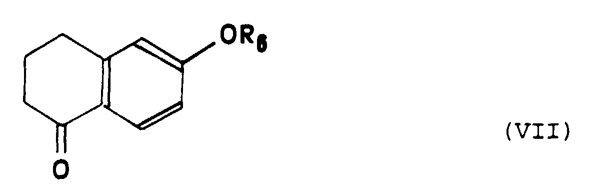 dans laquelle R₆ représente un atome d'hydrogène ou un groupe alkyle en C₁ à C₃, avec l'organolithien 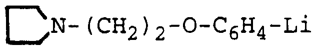 ou un organomagnésien de formule : 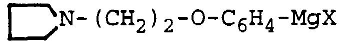 dans laquelle X représente un atome d'halogène choisi le chlore, le brome ou l'iode, qui conduit à la formation d'un alcool intermédiaire, suivie d'une réaction de deshydratation dudit alcool ;
b) on soumet le composé obtenu à l'étape a) à une réaction de bromation pour obtenir le composé de formule (VIII) : 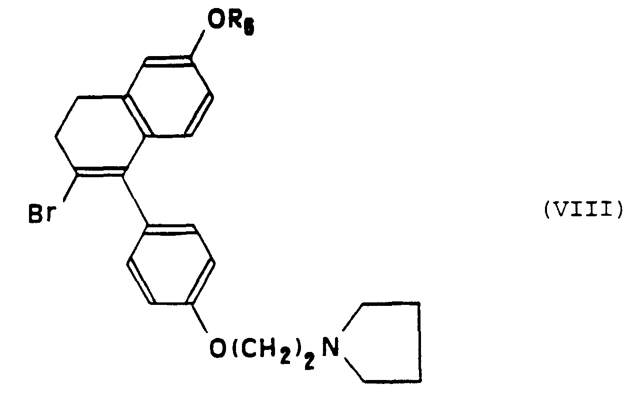
c) on soumet le composé de formule (VIII) ainsi obtenu à une réaction de couplage avec un composé organométallique de formule : Cp-M-(C₅H₄)-Y dans laquelle Cp représente un groupe cyclopentadiène, M représente un atome métallique choisi parmi le fer, le ruthénium ou l'osmium et Y représente un atome de lithium, un atome de cuivre, un groupe SnBu₃ ou un groupe HgCl.

La réaction de bromation à laquelle est soumis le composé de formule (VIII) peut être réalisée avec de l'acide bromhydrique en présence d'un solvant approprié comme par exemple la pyridine.

Les produits de formule générale (I) et leurs sels d'addition avec des acides acceptables du point de vue pharmacologique peuvent être administrés à l'homme en tant que médicaments, seuls ou sous la forme de compositions pharmaceutiques qui permettent une application entérale ou parentérale et qui contiennent, comme constituant actif, une dose efficace d'au moins un produit de formule (I) ou un sel d'addition d'acide d'un tel composé, avec en plus, des excipients et additifs inoffensifs pris parmi ceux dont on se sert ordinairement en pharmacie.

Les produits de formule générale (I) ainsi que leurs sels trouvent, en raison de leurs propriétés tumoristatiques et tumoricides, une application dans la préparation de médicaments à visée antitumorale notamment pour le traitement des cancers du sein oestrogéno-dépendants.

Outre les dispositions qui précèdent, l'Invention comprend encore d'autres dispositions qui ressortiront de la description qui suit, faite à titre d'exemples, et en référence aux dessins annexés dans lesquels :

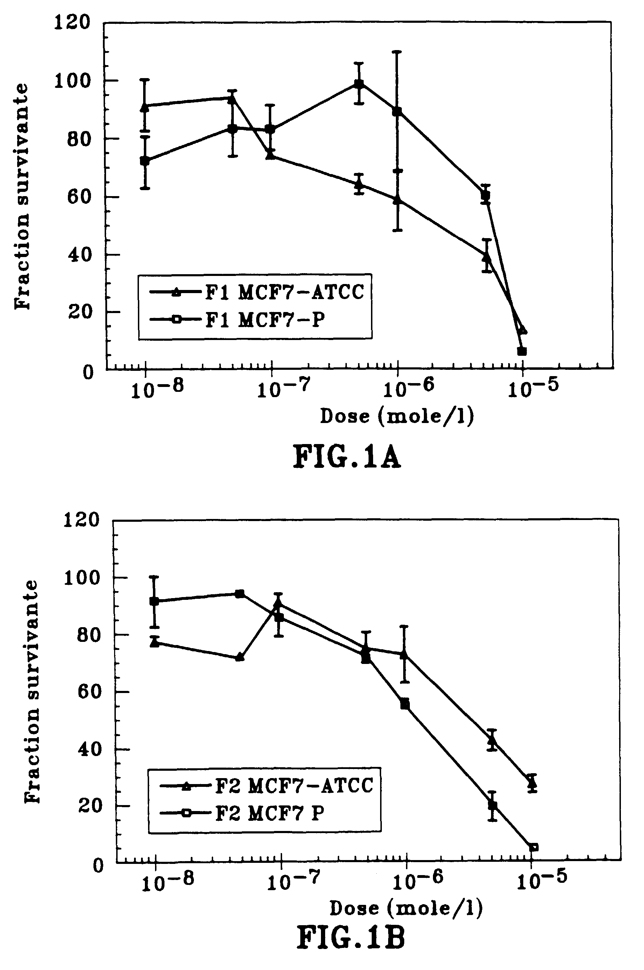

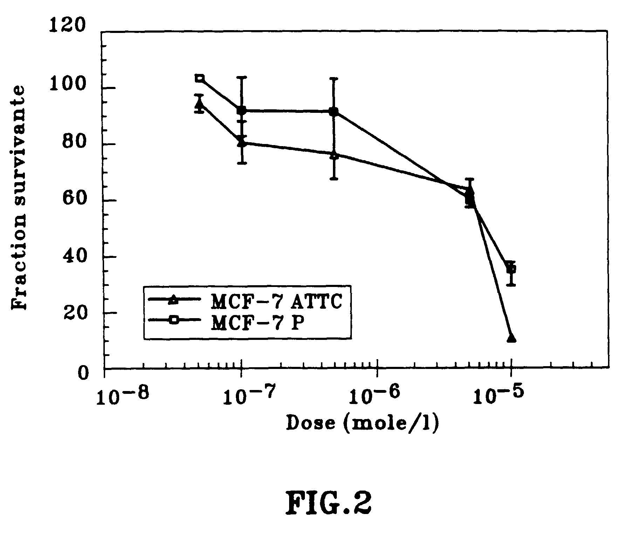

- les Figures 1A, 1B et 2 montrent les résultats des tests de cytotoxicité réalisés respectivement avec les deux isomères d'un dérivé métallocénique conforme à l'Invention et le tamoxifène.

Il doit être bien entendu, toutefois, que ces exemples sont donnés à titre d'illustrations de l'Invention dont ils ne constituent en aucune manière une limitation.

### EXEMPLE 1 : Préparation des isomères "trans" et "cis" du 1-[4-(2-diméthylaminoéthoxy) phényl]-1-(4-hydroxyphényl)-2-ferrocényl-1-butène

### 1.1 : Préparation du ferrocénylacétonitrile

8,00 g (20,8 mmol) d'iodure de ferrocénylméthyl-triméthyl ammonium (STREM) et 8 g de KCN (123 mmol) sont dissous dans 80 ml d'eau. Le mélange est placé dans un ballon surmonté d'un réfrigérant. On chauffe à reflux pendant 2 heures puis on laisse le mélange refroidir à la température ambiante. On extrait le produit par 3 fois 40 ml d'éther éthylique. La phase organique est lavée d'abord par 2 fois 30 ml d'eau puis séchée sur sulfate de magnésium. Après filtration et évaporation, on recueille 4,1 g de ferrocénylacétonitrile.
Formule chimique : C₁₂H₁₁NFe
Masse : 225
Rendement : 87,7%.

### 1.2 : Préparation de l'acide ferrocénylacétique

4,10 g (18,2 mmol) de ferrocénylacétonitrile sont dissous dans 80 ml d'éthanol. On dissout 10,08 g de KOH (180 mmol) dans 80 ml d'eau. On verse la solution de potasse dans la première solution et le mélange est porté à reflux pendant 4 heures. Le milieu réactionnel est concentré pour éliminer la majorité de l'éthanol. On extrait le produit par 3 fois 30 ml d'éther. La phase aqueuse est acidifiée par de l'acide chlorhydrique jusqu'à pH 1 et on extrait de nouveau par 4 fois 50 ml d'éther éthylique. Les phases organiques sont réunies, séchées sur sulfate de magnésium, filtrées puis évaporées. On obtient 4,20 g d'acide ferrocénylacétique sous forme d'un solide jaune.
Formule chimique : C₁₂H₁₂O₂Fe
Masse : 244
Rendement : 94,5%.
RMN ¹H (200 MHz, CDCl₃) : δ 4,23 (m, 2H, Cp substitué), 4,15 (s, 7H, Cp et Cp substitué), 3,42 (s, 2H, CH₂).

### 1.3 : Préparation du ferrocénylacétate d'éthyle

2,00 g (8,2 mmol) d'acide ferrocénylacétique sont dissous dans 50 ml d'éthanol. On ajoute 0,5 ml d'acide sulfurique concentré. Le mélange est porté à reflux pendant 1 heure. Après refroidissement, le milieu réactionnel est versé dans 100 ml d'eau glacée. On extrait le produit par 3 fois 40 ml d'éther. La phase éthérée est d'abord lavée à l'eau (2 fois 40 ml), puis séchée sur sulfate de magnésium, filtrée et évaporée. On obtient 2,22 g du ferrocénylacétate d'éthyle. Le rendement de la réaction est de 100%.
Formule chimique : C₁₄H₁₆O₂Fe
Masse : 272
RMN ¹H (200 MHz, CDCl₃) : δ 4,23 (m, 2H, Cp substitué), 4,18 (m, 2H, O-CH₂), 4,14 (s, 5H, Cp), 4,13 (m, 2H, Cp substitué), 3,34 (s, 2H, Cp-CH₂), 1,31 (t, 3H, J=7,12 Hz, Me).

### 1.4 : Préparation du 2-éthyl-2-ferrocénylacétate d'éthyle

2,22 g (8,2 mmol) de ferrocénylacétate d'éthyle sont dissous dans 25 ml de DMSO anhydre sous atmosphère argon. On ajoute 1,01 g (9 mmol) de t-BuOK. Après 5 minutes d'agitation, 1,5 g (9,6 mmol) d'iodoéthane sont rapidement ajoutés. L'agitation est poursuivie pendant 2 minutes puis le milieu réactionnel est versé dans 100 ml d'eau glacée. On extrait le produit par 3 fois 50 ml d'éther. La phase éthérée est lavée 2 fois par 50 ml d'eau, séchée sur sulfate de magnésium, filtrée puis évaporée. Le produit brut obtenu est chromatographié sur plaques de gel de silice 60GF₂₅₄ (MERCK 7730) d'1 mm d'épaisseur avec comme éluant un mélange éther/pentane 1:9.
On obtient 1,16 g de 2-éthyl-2-ferrocényl-acétate d'éthyle (Rf = 0,6) avec un rendement de 47%.
Formule chimique : C₁₆H₂₀O₂Fe
Masse : 300
RMN ¹H (200 MHz, CDCl₃) : δ 4,23 (m, 2H, Cp substitué), 4,16 (m, 2H, O-CH₂), 4,13 (s, 5H, Cp), 4,12 (m, 2H, Cp substitué), 3,19 (m, 1H, Fc-CH-), 1,77 (m, 2H, Fc-CH-CH₂), 1,35 (t, 3H, J=7,14 Hz, O-CH₂-CH₃), 0,92 (t, 3H, J=7,38 Hz, CH-CH₂-CH₃).

### 1.5 : Préparation du 1,1-Bis-(4-méthoxyphényl)-2-ferrocényl-1-butène

1,20 g (5,5 mmol) de 4-iodoanisole sont dissous dans 20 ml d'éther anhydre sous atmosphère argon. On refroidit la solution à 0°C puis on ajoute goutte-à-goutte 2,9 ml (4,6 mmol) d'une solution de n-butyllithium à 1,6 M. Après addition complète, le mélange est maintenu à 0°C pendant 15 minutes. On place dans l'ampoule à brome une solution de 2-éthyl-2-ferrocénylacétate d'éthyle (0,69 g, 2,3 mmol) dissous dans 10 ml d'éther anhydre. On ajoute goutte-à-goutte cette dernière solution à la première solution en maintenant la température à 0'C. L'agitation est maintenue pendant 30 minutes à 0'C et 2 heures à température ambiante. On ajoute ensuite une solution d'acide chlorhydrique 1N jusqu'à pH 1 et on chauffe le milieu réactionnel à reflux pendant 4 heures. Après refroidissement, on extrait le produit à l'éther. La phase éthérée est d'abord lavée à l'eau, séchée sur sulfate de magnésium, filtrée puis évaporée. Le brut obtenu est chromatographié sur plaques de gel de silice 60GF₂₅₄ avec comme éluant un mélange éther/pentane 1:9. On obtient 0,65 g du composé désiré (Rf = 0,45) avec un rendement de 62,5%.
Formule chimique : C₂₈H₂₈O₂Fe
Masse : 452
RMN ¹H (200 MHz, CDCl₃) : δ 7,12 et 6,16 (dd, 4H, J=8,4 Hz, C₆H₄), 6,98 et 6,87 (dd, 4H, J=8,4 Hz, C₆H₄), 4,14 (s, 5H, Cp), 4,10 (m, 2H, Cp substitué), 3,95 (m, 2H, Cp substitué), 3,82 et 3,78 (s, s, 3H, 3H, O-Me), 2,56 (q, 2H, J=7,4 Hz, -CH₂-CH₃), 1,02 (t, 3H, J=7,3 Hz, -CH₂-CH₃).

### 1.6 : Préparation du 1,1-Bis-(4-hydroxyphényl)-2-ferrocényl-1-butène

0,55 g (1,28 mmol) de 1,1-Bis-(4-méthoxyphényl)-2-ferrocényl-1-butène sont dissous dans 5 ml de dichlorométhane sous atmosphère d'argon. On refroidit la solution à -78°C puis on ajoute 0,3 ml (2,84 mmol) de BBr₃. Après addition complète, on retire le bain froid et on maintient l'agitation pendant 30 minutes. On verse ensuite le milieu réactionnel dans de l'eau glacée. Après 10 minutes d'agitation, on ajoute du NaCl jusqu'à saturation et on extrait le produit au dichlorométhane (4 fois 40 ml). La phase organique est d'abord lavée à l'eau, séchée sur sulfate de magnésium, filtrée puis évaporée. Le produit brut obtenu est chromatographié sur plaques de gel de silice 60GF₂₅₄ avec comme éluant un mélange éther/pentane 3:2.
On obtient 0,43 g du composé désiré (Rf = 0,4 ; rendement = 79%).
Formule chimique : C₂₆H₂₄O₂Fe
Masse : 424
RMN ¹H (200 MHz, CDCl₃) : δ 7,07 et 6,68 (d, d, 4H, J=8,6 Hz, C₆H₄), 6,92 et 6,80 (d, d, 4H, J=8,5 Hz, C₆H₄), 4,88 et 4,42 (s, s, H, H, 20H), 4,11 (s, 5H, Cp), 4,08 (m, 2H, Cp substitué), 3,91 (m, 2H, Cp substitué), 2,58 (q, 2H, J=7,5 Hz, -CH₂-CH₃), 1,02 (t, 3H, J=7,5 Hz, -CH₂-CH₃).

### 1.7 : Obtention des isomères "trans" et "cis" du 1-[4-(2-diméthylaminoéthoxy) phényl]-1-(4-hydroxyphényl)-2-ferrocényl-1-butène

On prépare d'abord une solution d'éthanolate de sodium en faisant réagir 0,120 g (5,2 mmol) de sodium avec 20 ml d'éthanol. On ajoute ensuite à cette solution 0,450 g (1,06 mmol) de 1,1-Bis-(4-hydroxyphényl)-2-ferrocényl-1-butène dissous dans 10 ml d'éthanol. Après 1 heure d'agitation à 80°C, on ajoute 0,302 g (2,1 mmol) de chlorure de de 2-diméthyl-aminoéthyle hydrochlorure et on chauffe à reflux pendant 3 heures. On laisse ensuite la solution refroidir jusqu'à la température ambiante. On hydrolyse avec 100 ml d'eau et on extrait le produit à l'éther (4 fois 50 ml). La phase organique est d'abord lavée à l'eau, séchée sur sulfate de magnésium, filtrée puis évaporée. Le produit brut obtenu est chromatographié sur plaques de gel de silice 60GF₂₅₄ avec comme éluant un mélange (C₂H₅)₃N/chloroforme 1:9.

On obtient d'abord 0,280 g du mélange de l'isomère "trans" et de l'isomère "cis" du 1-[4-(2-diméthylaminoéthoxy) phényl]-1-(4-hydroxyphényl)-2-ferrocényl-1-butène (Rendement = 53%).

La deuxième fraction, plus polaire, correspond au 1,1-Bis [4-(2-diméthylaminoéthoxy) phényl]-2-ferrocényl-1-butène (0,050g ; Rendement = 9,5%).

### 1.8 : Séparation des isomères "trans" et "cis " du 1-[4-(2-diméthylaminoéthoxy) phényl]-1-(4-hydroxyphényl)-2-ferrocényl-1-butène

Le mélange des deux isomères précédemment obtenu est dissous dans un mélange éther/hexane (5:1) et la solution obtenue est laissée dans un réfrigérateur pendant 1 nuit.

La solubilité dans l'éther de l'isomère "cis" étant plus faible que celle de l'isomère "trans", l'isomère "cis" cristallise en premier après une évaporation douce du solvant. On isole les cristaux de l'isomère "cis" et les eaux-mères sont mises à refroidir dans la partie congélateur (-15'C) du réfrigérateur pendant un jour. L'isomère "trans" cristallise alors à son tour sous forme de fines aiguilles.

### EXEMPLE 2 : Caractérisation des isomères "trans" et "cis" du 1-[4-(2-diméthylaminoéthoxy) phényl]-1-(4-hydroxy phényl)-2-ferrocényl-1-butène

### 2.1 : Formule chimique et masse molaire

Formule chimique : C₃₀H₃₃O₂NFe

Masse molaire : 495,4

### 2.2 : Données spectrométriques RMN ¹H

Les spectres de RMN ¹H des deux isomères "trans" et "cis" ont été enregistrés sur un spectromètre BRUKER AC200 MHz dans du DMSOd6 (JANSSEN), en raison de ce que ce solvant n'entraîne pas une isomérisation des deux isomères.

Les valeurs RMN ¹H des deux isomères sont les suivantes :
Isomère "trans" :
   δ:9.34 (s, 1H, OH), 6.97 et 6.71 (d, d, 4H, J=8,7 Hz, C₆H₄-OH), 6.89 et 6.80 (d, d, 4H, J=8.5 Hz, C₆H₄-CH₂), 4.11 (s, 5H, Cp), 4.07 (m, 2H, Cp substitué), 3.99 (t, 2H, J=6,0 Hz, O-CH₂), 3.80 (m, 2H, Cp substitué), 2.60 (t, 2H, J=6.0 Hz, N-CH₂), 2.49 (masqué par le signal de DMSO, 2H, -CH₂-CH₃), 2.20 (s, 6H, NMe₂), 0.98 (t, 3H, J=7.3 Hz, -CH₂-CH₃).
Isomère "cis" :
   δ:9.29 (s, 1H, OH), 7.08 et 6.89 (d,d, 4H, J=8,5 Hz, C₆H₄-OCH₂), 6.80 et 6.63 (d,d, 4H, J=8.4 Hz, C₆H₄-OH), 4.11 (s, 5H, Cp), 4.08 (m, 2H, (t, 2H, J=6,0 Hz, N-CH₂), 2.49 (masqué par le signal de DMSO, 2H, -CH₂-CH₃), 2.23 (s, 6H, NMe₂), 0.98 (t, 3H, J=7.3 Hz, -CH₂-CH₃).

### 2.3 : Points de fusion

Les points de fusion des isomères "trans" et "cis" ont été déterminés à l'aide d'un banc de Kofler. Ils sont respectivement de 93-94°C pour l'isomère "trans" et de 181'C pour l'isomère "cis".

### EXEMPLE 3 : Activité cytotoxique des isomères "trans" et "cis" du 1-[4-(2-diméthylaminoéthoxy) phényl]-1-(4-hydroxyphényl)-2-ferrocényl-1-butène

L'évaluation de la cytotoxicité des isomères "trans" et "cis" du 1-[4-(2-diméthylaminoéthoxy) phényl]-1-(4-hydroxyphényl)-2-ferrocényl-1-butène a fait l'objet de tests sur des lignées de cellules humaines dérivées d'une effusion pleurale d'un adénocarcinome mammaire (lignée MCF-7 ATCC HTB) ainsi que de l'un de ses variants (lignée MCF-7P).

Ces cellules ont été préalablement cultivées dans du milieu Dulbecco MEM additionné de 10% de sérum de veau foetal et en atmosphère humide (90% d'humidité relative) comprenant 5% de CO₂. En phase exponentielle de croissance, ces cellules ont été trypsinées et repiquées dans des plaques de 24 puits de 1 ml à raison de 2.10⁵ cellules par puits.

Après 3 jours d'incubation, les composés à tester ont été ajoutés aux doses appropriées et les plaques ont été mises à incuber pendant 5 jours.

A l'issue de cette incubation, la viabilité cellulaire des cultures a été déterminée à l'aide du test dit "MTT" consistant à ajouter dans chaque puits 20 µl d'une solution contenant 5 mg de bromure de (diméthyl-4,5-thiazol-2-yl)diphényl-2,5-tétrazolium (MTT) dans 1 ml de tampon PBS, et à laisser incuber les plaques pendant 1 heure à 37°C dans des conditions atmosphériques identiques à celles définies ci-dessus. Le MTT est métabolisé dans les cellules vivantes en un composé bleu insoluble (formazan).

A l'issue de cette incubation, les puits sont lavés à l'aide de PBS pour éliminer le MTT et les cristaux résiduels de formazan sont repris dans 1 ml de DMSO. La concentration en formazan pour chaque puits est lue par photométrie à une longueur d'onde de 550 nm et comparée à un témoin correspondant à du milieu incubé en présence de MTT mais en l'absence de cellules.

Les Figures 1A et 1B montrent les résultats des tests de cytoxicité réalisés respectivement avec l'isomère "cis" (Figure lA) et l'isomère "trans" (Figure 1B) du 1-[4-(2-diméthylaminoéthoxy) phényl]-1-(4-hydroxyphényl)-2-ferrocényl-1-butène sur les deux lignées cellulaires indiquées ci-avant. Ces résultats sont exprimés en fractions de cellules survivantes en fonction des doses du composé testé.

A titre de comparaison, la Figure 2 montre les résultats obtenus en effectuant les mêmes tests et dans les mêmes conditions avec le tamoxifène.

Le Tableau 1 ci-après présente les doses inhibitrices 50 (IC₅₀) exprimées en moles et calculées sur les points des Figures 1A, 1B et 2 compris entre 5.10⁻⁷ et 10⁻⁵ moles/1.

**TABLEAU 1 :**

| **COMPOSE TESTE** | **IC**_{**50**} **(µmole)** | |
|---|---|---|
| | **MCF7 ATCC** | **MCF7-P** |
| Dérivé "cis" | 3,4 | 5,6 |
| Dérivé "trans" | 4,9 | 2 |
| Tamoxifène | 6,4 | 7,2 |

Plus la dose inhibitrice 50 est faible, plus l'activité cytotoxique est élevée et les résultats exprimés dans le Tableau 1 montrent que les isomères "cis" et "trans" du 1-[4-(2-diméthylaminoéthoxy) phényl]-1-(4-hydroxy-phényl)-2-ferrocényl-1-butène présentent, sur les deux lignées cellulaires testées, une activité cytotoxique supérieure à celle du tamoxifène.

Ainsi que cela ressort de ce qui précède, l'Invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente Invention.

## Revendications

1. Composés de formule générale (I) : 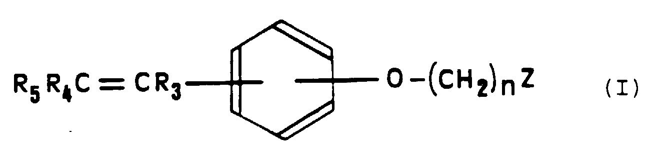 dans laquelle :
le groupe -O(CH₂)ₙZ se trouve en position méta ou para par rapport au groupe -CR₃=CR₄R₅,
n représente un nombre entier compris entre 2 et 10,
Z représente un coordinat basique ou polaire choisi parmi les groupes -NR₁R₂ dans lesquels R₁ et R₂, identiques ou différents, représentent un groupe alkyle en C₁ à C₆ éventuellement substitué par un ou plusieurs atomes d'halogène, les groupes hétérocycliques renfermant un atome d'azote, ou les groupes -OR₁, -SR₁, -SOR₁ ou -SO₂R₁ dans lesquels R₁ a la même signification que précédemment, et dans laquelle :
* soit R₃ représente un groupe phényle éventuellement substitué par un ou plusieurs atomes d'hydrogène ou atomes d'halogène ou groupes hydroxyle ou groupes alkyle ou alcoxy en C₁ à C₃ et R₅ représente un groupe métallocène, tandis que R₄ représente un atome d'hydrogène ou un atome d'halogène ou un groupe NO₂ ou un groupe chloroéthyle ou un groupe CN ou encore un groupe alkyle en C₁ à C₃ dont le carbone terminal peut éventuellement être lié au carbone situé en position 2 du groupe phényle de R₃,
* soit R₃ représente un groupe métallocène et R₅ représente un groupe phényle éventuellement substitué par un ou plusieurs atomes d'hydrogène ou atomes d'halogène ou groupes hydroxyle ou groupes alkyle ou alcoxy en C₁ à C₃, tandis que R₄ représente un atome d'hydrogène ou un atome d'halogène ou un groupe NO₂ ou un groupe chloroéthyle ou un groupe CN ou encore un groupe alkyle en C₁ à C₃,
sous toutes leurs formes stéréoisomères ainsi que leurs sels.

2. Composés selon la Revendication 1, caractérisés en ce que le groupe métallocène répond à la formule générale (II) : 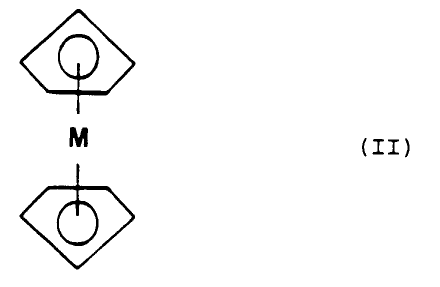 dans laquelle M représente un atome métallique du groupe VIII de la Classification Périodique des éléments chimiques, ledit atome pouvant être éventuellement sous une forme oxydée.

3. Composés selon la Revendication 1, caractérisés en ce que le groupe métallocène répond à la formule générale (III) : 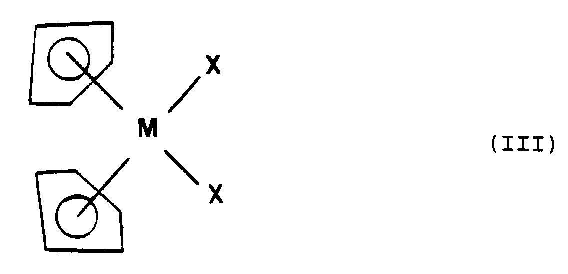 dans laquelle :
M représente un atome métallique choisi parmi le titane, le vanadium, le nobium, le hafnium ou le molybdène,
X représente un atome d'halogène choisi parmi le chlore, le brome ou l'iode.

4. Composés selon la Revendication 1 ou la Revendication 2, caractérisés en ce qu'ils répondent à la formule particulière (I-a) : 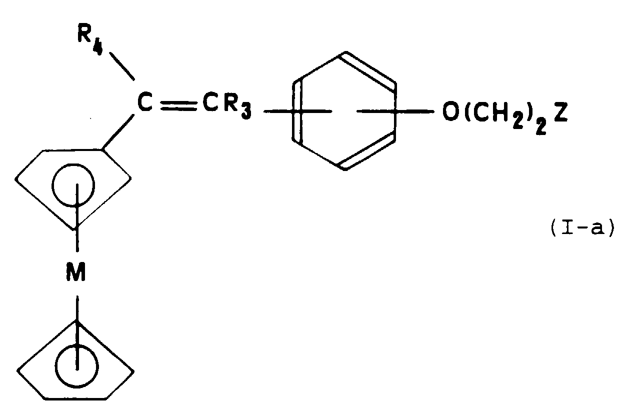 dans laquelle :
le groupe -O(CH₂)₂Z se trouve en position para par rapport au groupe -CR₃=CR₄R₅,
Z représente un groupe -NR₁R₂ dans lequel R₁ et R₂, identiques ou différents, représentent un groupe méthyle ou un groupe éthyle ou bien Z représente un groupe N-pyrrolidino,
R₃ représente un groupe phényle éventuellement substitué par un groupe hydroxyle ou par un groupe méthoxy,
R₄ représente un atome d'hydrogène ou un atome d'halogène ou un groupe NO₂ ou un groupe chloroéthyle ou un groupe CN ou encore un groupe éthyle, et
M représente un atome métallique choisi parmi le fer, le ruthénium ou l'osmium, éventuellement sous sa forme oxydée.

5. Composés selon la Revendication 4, caractérisés en ce que R₁ et R₂ représentent un groupe méthyle, R₃ représente un groupe phényle substitué en para par un groupe hydroxyle et R₄ représente un groupe éthyle, ces composés répondant à la formule particulière (I-a1).

6. Composé selon la Revendication 5, caractérisé en ce qu'il est le 1-[4-(2-diméthylaminoéthoxy) phényl]-1-(4-hydroxyphényl)-2-ferrocényl-1-butène.

7. Composé selon la Revendication 6, caractérisé en ce qu'il est l'isomère "trans" du 1-[4-(2-diméthylaminoéthoxy) phényl]-1-(4-hydroxyphényl)-2-ferrocényl-1-butène.

8. Composés selon la Revendication 4, caractérisés en ce que R₁ et R₂ représentent un groupe méthyle, R₃ représente un groupe phényle et R₄ représente un groupe éthyle, ces composés répondant à la formule particulière (I-a2).

9. Composé selon la Revendication 8, caractérisé en ce qu'il est le 1-[4-(2-diméthylaminoéthoxy) phényl]-1-phényl-2-ferrocényl-1-butène.

10. Composés selon la Revendication 1 ou la Revendication 2, caractérisés en ce que :
le groupe -O(CH₂)ₙZ se trouve en position para par rapport au groupe -CR₃=CR₄R₅,
n est égal à 2 et Z représente un groupe N-pyrrolidino,
R₃ représente un groupe phényle substitué en para par un groupe hydroxyle ou alcoxy en C₁ à C₃, tandis que R₄ représente un groupe alkyle en C₂ dont le carbone terminal est lié au carbone situé en position 2 du groupe phényle de R₃,
M représente un atome métallique choisi parmi le fer, le ruthénium ou l'osmium, éventuellement sous sa forme oxydée,
ces composés répondant à la formule particulière (I-b).

11. Procédé de préparation d'un composé selon l'une quelconque des Revendications 1 à 10, caractérisé en ce que :
a) on fait réagir un composé de formule générale (IV) : 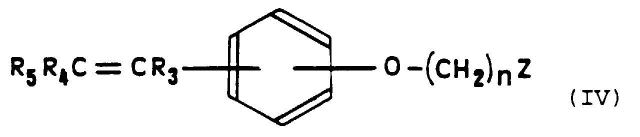 dans laquelle :
le groupe -O(CH₂)ₙZ est en position méta ou para par rapport au groupe -CR₃=CR₄R₅,
n représente un nombre entier compris entre 2 et 10,
Z représente un coordinat basique ou polaire choisi parmi les groupes -NR₁R₂ dans lesquels R₁ et R₂, identiques ou différents, représentent un groupe alkyle en C₁ à C₆ éventuellement substitué par un ou plusieurs atomes d'halogène, les groupes hétérocycliques renfermant un atome d'azote, ou les groupes -OR₁, -SR₁, -SOR1 ou -SO₂R1 dans lesquels R₁ a la même signification que précédemment, et dans laquelle :
* soit R₃ représente un groupe phényle éventuellement substitué par un ou plusieurs atomes d'hydrogène ou atomes d'halogène ou groupes hydroxyle ou groupes alkyle ou alcoxy en C₁ à C₃ et R₅ représente un atome de brome, tandis que R₄ représente un atome d'hydrogène ou un atome d'halogène ou un groupe NO₂ ou un groupe chloroéthyle ou un groupe CN ou encore un groupe alkyle en C₁ à C₃ dont le carbone terminal peut éventuellement être lié au carbone situé en position 2 du groupe phényle de R₃,
* soit R₃ représente un atome de brome et R₅ représente un groupe phényle éventuellement substitué par un ou plusieurs atomes d'hydrogène ou atomes d'halogène ou groupes hydroxyle ou groupes alkyle ou alcoxy en C₁ à C₃, tandis que R₄ représente un atome d'hydrogène ou un atome d'halogène ou un groupe NO₂ ou un groupe chloroéthyle ou un groupe CN ou encore un groupe alkyle en C₁ à C₃, avec un sel métallique de cyclopentadiényle pour obtenir la substitution de l'atome de brome par un groupe cyclopentadiène ;
b) on fait réagir le composé obtenu à l'étape a) avec une base forte pour obtenir la formation d'un anion cyclopentadiènyle,
c) on fait réagir le composé obtenu à l'étape b) avec un composé organométallique de formule : Cp-M-(CO)₂-X dans laquelle Cp représente un groupe cyclopentadiène, M représente un atome métallique du groupe VIII de la Classification Périodique des éléments chimiques et X représente un atome d'halogène choisi parmi le chlore, le brome ou l'iode,
ou avec un composé organométallique de formule : Cp-M-Xm dans laquelle Cp et X ont la même signification que précédemment, M est un atome métallique choisi parmi le titane, le vanadium, le nobium, le hafnium ou le molybdène et m est un nombre entier égal à 3 ou 4.

12. Procédé de préparation d'un composé de formule particulière (I-al) selon l'une quelconque des Revendications 5 à 7, caractérisé en ce que :
a) on soumet un ester métallocénique de formule (V) : 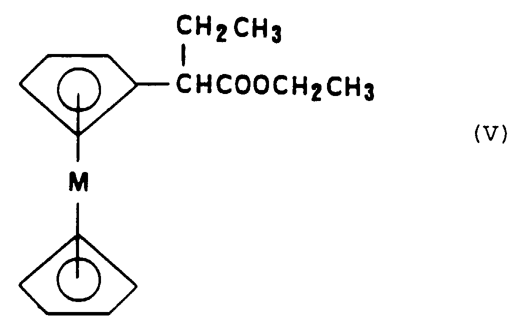 dans laquelle M représente un atome métallique choisi parmi le fer, le ruthénium ou l'osmium, à une réaction d'addition avec l'organolithien CH₃-O-C₆H₄-Li ou un organomagnésien de formule : CH₃-O-C₆H₄-MgX dans laquelle X représente un atome de chlore, de brome ou d'iode, qui conduit à la formation d'un alcool intermédiaire, suivie d'une réaction de deshydratation dudit alcool,
b) on soumet le composé obtenu à l'étape a) à une réaction de déméthylation,
c) on soumet le composé obtenu à l'étape b) à une réaction d'alkylation par l'éthanolate de sodium et l'hydrochlorure de chlorure de 2-diméthylaminoéthyle.

13. Procédé de préparation selon la Revendication 12, caractérisé en ce qu'il comprend préalablement la préparation de l'ester métallocénique de formule (V) par estérification de l'acide métallocénylacétique correspondant, suivie d'une réaction d'alkylation du carbone en position α du cycle métallocényle dudit ester.

14. Procédé de préparation selon la Revendication 12 ou la Revendication 13, caractérisé en ce qu'il comprend de plus une étape de séparation des isomères "cis" et "trans" du composé de formule particulière (I-a1) obtenu.

15. Procédé de préparation des composés de formule particulière (I-a2) selon la Revendication 8 ou la Revendication 9, caractérisé en ce que :
a) on fait réagir un chlorure d'acide métallocénique de formule (VI) : 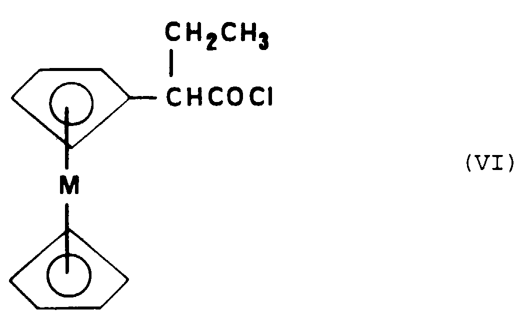 dans laquelle M représente un atome métallique choisi parmi le fer, le ruthénium ou l'osmium, avec du diphényle de zinc pour obtenir la substitution de l'atome de chlore par un groupe phényle,
b) on soumet le composé obtenu à l'étape a) à une réaction d'addition avec l'organolithien (CH₃)₂-N-(CH₂)₂-O-C₆H₄-Li ou un organomagnésien de formule : (CH₃)₂-N-(CH₂)₂-O-C₆H₄-MgX dans laquelle X représente un atome d'halogène choisi parmi le chlore, le brome ou l'iode, qui conduit à la formation d'un alcool intermédiaire, suivie d'une réaction de deshydratation dudit alcool.

16. Procédé de préparation selon la Revendication 15, caractérisé en ce qu'il comprend, en outre, une étape de séparation des isomères "cis" et "trans" du composé de formule particulière (I-a2) obtenu.

17. Procédé de préparation d'un composé de formule particulière (I-b) selon la Revendication 10, caractérisé en ce que :
a) on fait réagir une tétralone de formule (VII) : 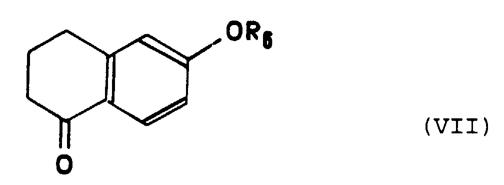 dans laquelle R₆ représente un atome d'hydrogène ou un groupe alkyle en C₁ à C₃, avec l'organolithien 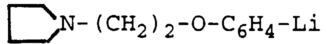 ou un organomagnésien de formule: 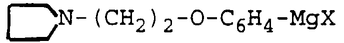 dans laquelle X représente un atome d'halogène choisi parmi le chlore, le brome ou l'iode, qui conduit à la formation d'un alcool intermédiaire, suivie d'une réaction de deshydratation dudit alcool,
b) on soumet le composé obtenu à l'étape a) à une réaction de bromation pour obtenir le composé de formule (VIII) : 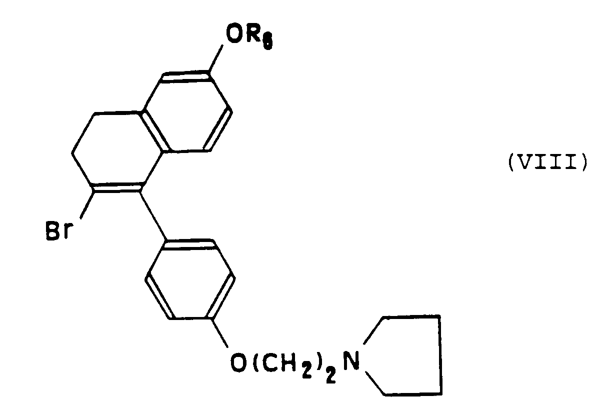
c) on soumet le composé de formule (VIII) ainsi obtenu à une réaction de couplage avec un composé organométallique de formule : Cp-M-(C₅H₄)-Y dans laquelle Cp représente un groupe cyclopentadiène, M représente un atome métallique choisi parmi le fer, le ruthénium ou l'osmium et Yreprésente un atome de lithium, un atome de cuivre, un groupe SnBu₃ ou un groupe HgCl.

18. Composition pharmaceutique, caractérisée en ce qu'elle contient au moins l'un des composés de formule (I) selon l'une quelconque des Revendications 1 à 10 ou l'un de ses sels, éventuellement associé à un véhicule pharmacologiquement acceptable.

19. Utilisation d'un composé de formule (I) ou de l'un de ses sels selon l'une quelconque des Revendications 1 à 10 pour l'obtention d'un médicament antitumoral.

## Patentansprüche

1. Verbindungen der allgemeinen Formel (I): 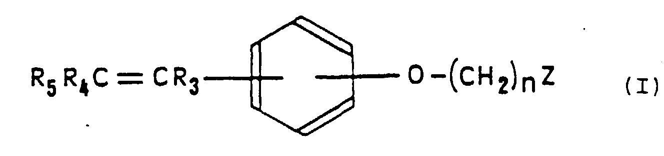 worin:
sich die Gruppe -O(CH₂)ₙZ in der Meta- oder Paraposition bezüglich der Gruppe -CR₃=CR₄R₅ befindet,
n für eine ganze Zahl zwischen 2 und 10 steht,
Z für einen basischen oder polaren Liganden steht, ausgewählt aus den Gruppen -NR₁R₂, worin R₁ und R₂, die identisch oder verschieden sind, für eine gegebenenfalls durch ein oder mehrere Halogenatome substituierte C₁-C₆-Alkylgruppe stehen, den heterocyclischen Gruppen, die ein Stickstoffatom enthalten, oder den Gruppen -OR₁, -SR₁, -SOR₁ oder-SO₂R₁, in denen R₁ die gleiche Bedeutung wie zuvor hat, und worin:
* entweder R₃ für eine Phenylgruppe steht, die gegebenenfalls durch ein oder mehrere Wasserstoffatome oder Halogenatome oder Hydroxylgruppen oder C₁-C₃-Alkyl- oder Alkoxygruppen substituiert ist, und R₅ für eine Metallocengruppe steht, während R₄ für ein Wasserstoffatom oder ein Halogenatom oder eine NO₂-Gruppe oder eine Chlorethylgruppe oder eine CN-Gruppe oder auch eine C₁-C₃-Alkylgruppe, deren terminales Kohlenstoffatom gegebenenfalls mit dem Kohlenstoffatom in der Position 2 der Phenylgruppe von R₃ verbunden sein kann, steht,
* oder R₃ für eine Metallocengruppe steht und R₅ für eine Phenylgruppe steht, die gegebenenfalls durch ein oder mehrere Wasserstoffatome oder Halogenatome oder Hydroxylgruppen oder C₁-C₃-Alkyl- oder Alkoxygruppen substituiert ist, während R₄ für ein Wasserstoffatom oder ein Halogenatom oder eine NO₂-Gruppe oder eine Chlorethylgruppe oder eine CN-Gruppe oder eine C₁-C₃-Alkylgruppe steht,
in allen ihren stereoisomeren Formen sowie ihre Salze.

2. Verbindungen nach Anspruch 1, dadurch **gekennzeichnet** , daß die Metallocengruppe der allgemeinen Formel (II) entspricht: 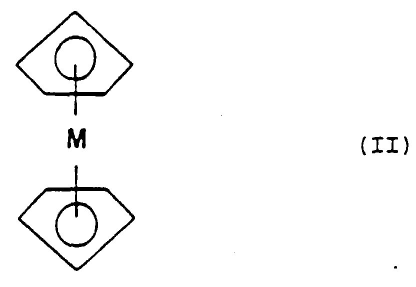 in der M für ein Metallatom der Gruppe VIII des Periodensystems der Elemente steht, wobei dieses Atom gegebenenfalls in einer oxidierten Form vorliegen kann.

3. Verbindungen nach Anspruch 1, dadurch **gekennzeichnet** , daß die Metallocengruppe der allgemeinen Formel (III) entspricht: 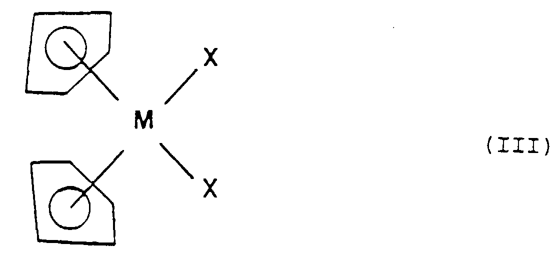 worin:
M für ein Metallatom, ausgewählt aus Titan, Vanadium, Niob, Hafnium oder Molybdän, steht,
X für ein Halogenatom, ausgewählt aus Chlor, Brom oder Iod, steht.

4. Verbindungen nach Anspruch 1 oder Anspruch 2, dadurch **gekennzeichnet,** daß sie der speziellen Formel (I-a) entsprechen: 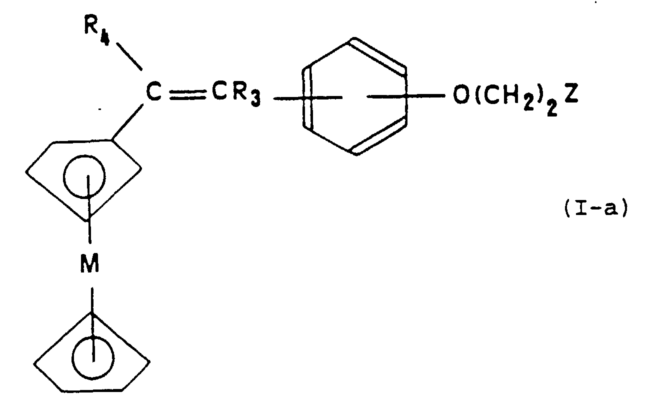 worin:
sich die Gruppe -O(CH₂)₂Z in der Paraposition bezüglich der Gruppe -CR₃=CR₄R₅ befindet,
Z für eine Gruppe -NR₁R₂ steht, in der R₁ und R₂, die identisch oder verschieden sind, für eine Methylgruppe oder eine Ethylgruppe stehen, oder Z für eine N-Pyrrolidinogruppe steht,
R₃ für eine Phenylgruppe steht, die gegebenenfalls durch eine Hydroxylgruppe oder eine Methoxygruppe substituiert ist,
R₄ für ein Wasserstoffatom oder ein Halogenatom oder eine NO₂-Gruppe oder eine Chlorethylgruppe oder eine CN-Gruppe oder auch eine Ethylgruppe steht, und
M für ein Metallatom, ausgewählt aus Eisen, Ruthenium oder Osmium, steht, das gegebenenfalls in seiner oxidierten Form vorliegt.

5. Verbindungen nach Anspruch 4, dadurch **gekennzeichnet** , daß R₁ und R₂ für eine Methylgruppe stehen, R₃ für eine Phenylgruppe steht, die in der Paraposition durch eine Hydroxylgruppe substituiert ist, und R₄ für eine Ethylgruppe steht, wobei diese Verbindungen der speziellen Formel (I-a1) entsprechen.

6. Verbindung nach Anspruch 5, dadurch **gekennzeichnet** , daß sie 1-[4-(2-Dimethylaminoethoxy)-phenyl]-1-(4-hydroxyphenyl)-2-ferrocenyl-1-buten ist.

7. Verbindung nach Anspruch 6, dadurch **gekennzeichnet** , daß sie das trans-Isomere von 1-[4-(2-Dimethylaminoethoxy)phenyl]-1-(4-hydroxyphenyl)-2-ferrocenyl-1-buten ist.

8. Verbindungen nach Anspruch 4, dadurch **gekennzeichnet** , daß R₁ und R₂ für eine Methylgruppe stehen, R₃ für eine Phenylgruppe steht und R₄ für eine Ethylgruppe steht, wobei diese Verbindungen der speziellen Formel (I-a2) entsprechen.

9. Verbindung nach Anspruch 8, dadurch **gekennzeichnet** , daß sie 1-[4-(2-Dimethylaminoethoxy)-phenyl]-1-phenyl-2-ferrocenyl-1-buten ist.

10. Verbindungen nach Anspruch 1 oder Anspruch 2, dadurch **gekennzeichnet,** daß:
sich die Gruppe -O(CH₂)ₙZ in der Paraposition bezüglich der Gruppe -CR₃=CR₄R₅ befindet,
n gleich 2 ist und Z für eine N-Pyrrolidiongruppe steht, R₃ für eine Phenylgruppe steht, die in der Paraposition durch eine Hydroxyl- oder C₁-C₃-Alkoxygruppe substituiert ist, während R₄ für eine C₂-Alkylgruppe steht, deren terminales Kohlenstoffatom mit dem Kohlenstoffatom in Position 2 der Phenylgruppe von R₃ verbunden ist,
M für ein Metallatom, ausgewählt aus Eisen, Ruthenium oder Osmium, steht, das gegebenenfalls in seiner oxidierten Form vorliegt,
wobei diese Verbindungen der speziellen Formel (I-b) entsprechen.

11. Verfahren zur Herstellung einer Verbindung nach einem der Ansprüche 1 bis 10, dadurch **gekennzeichnet**, daß :
a) man eine Verbindung der allgemeinen Formel (IV) umsetzt: 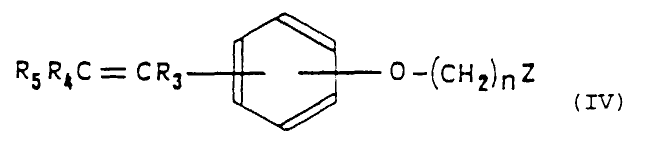 worin:
sich die Gruppe -O(CH₂)ₙZ in der Meta- oder Paraposition bezüglich der Gruppe -CR₃=CR₄R₅ befindet,
n für eine ganze Zahl zwischen 2 und 10 steht,
Z für einen basischen oder polaren Liganden steht, ausgewählt aus den Gruppen -NR₁R₂, worin R₁ und R₂, die identisch oder verschieden sind, für eine gegebenenfalls durch ein oder mehrere Halogenatome substituierte C₁-C₆-Alkylgruppe stehen, den heterocyclischen Gruppen, die ein Stickstoffatom enthalten, oder den Gruppen -OR₁, -SR₁, -SOR₁ oder -SO₂R₁, in denen R₁ die gleiche Bedeutung wie zuvor hat, und worin:
* entweder R₃ für eine Phenylgruppe steht, die gegebenenfalls durch ein oder mehrere Wasserstoffatome oder Halogenatome oder Hydroxylgruppen oder C₁-C₃-Alkyl- oder Alkoxygruppen substituiert ist, und R₅ für ein Bromatom steht, während R₄ für ein Wasserstoffatom oder ein Halogenatom oder eine NO₂-Gruppe oder eine Chlorethylgruppe oder eine CN-Gruppe oder auch eine C₁-C₃-Alkylgruppe, deren terminales Kohlenstoffatom gegebenenfalls mit dem Kohlenstoffatom in der Position 2 der Phenylgruppe von R₃ verbunden sein kann, steht,
* oder R₃ für ein Bromatom steht und R₅ für eine Phenylgruppe steht, die gegebenenfalls durch ein oder mehrere Wasserstoffatome oder Halogenatome oder Hydroxylgruppen oder C₁-C₃-Alkyl- oder Alkoxygruppen substituiert ist, während R₄ für ein Wasserstoffatom oder ein Halogenatom oder eine NO₂-Gruppe oder eine Chlorethylgruppe oder eine CN-Gruppe oder eine C₁-C₃-Alkylgruppe steht,
mit einem Metallsalz des Cyclopentadiens, um die Substitution des Bromatoms durch eine Cyclopentadienylgruppe zu erreichen;
b) man die in Schritt a) erhaltene Verbindung mit einer starken Base reagieren läßt, um die Bildung eines Cyclopentadienylanions zu ereichen,
c) man die in Schritt b) erhaltene Verbindung reagieren läßt mit einer metallorganischen Verbindung der Formel: Cp-M-(CO)₂-X, in der Cp für eine Cyclopentadiengruppe steht, M für ein Metallatom der Gruppe VIII des Periodensystems der Elemente steht und X für ein Halogenatom, ausgewählt aus Chlor, Brom oder Iod, steht,
oder mit einer metallorganischen Verbindung der Formel: Cp-M-Xₘ, in der Cp und X die gleiche Bedeutung wie zuvor haben, M ein Metallatom, ausgewählt aus Titan, Vanadium, Niob, Hafnium oder Molybdän, ist, und m eine ganze Zahl gleich 3 oder 4 ist.

12. Verfahren zur Herstellung einer Verbindung der speziellen Formel (I-a1) nach einem der Ansprüche 5 bis 7, dadurch **gekennzeichnet**, daß:
a) man einen Metallocenester der Formel (V): 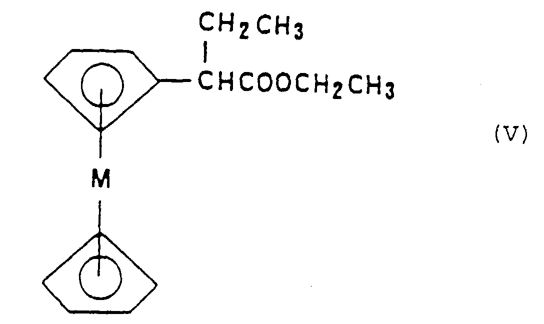 worin M für ein Metallatom, ausgewählt aus Eisen, Ruthenium oder Osmium, steht, einer Additionsreaktion mit der Organolithiumverbindung CH₃-O-C₆H₄-Li oder einer Organomagnesiumverbindung der Formel: CH₃-O-C₆H₄-MgX, in der X für ein Chlor-, Brom- oder Iodatom steht, unterwirft, die zur Bildung eines intermediären Alkohols führt, gefolgt von einer Dehydratisierungsreaktion des genannten Alkohols,
b) man die in Schritt a) erhaltene Verbindung einer Demethylierungsreaktion unterwirft,
c) man die in Schritt b) erhaltene Verbindung einer Alkylierungsreaktion durch Natriumethanolat und das Hydrochlorid des 1-Chlor-2-dimethylaminoethans unterwirft.

13. Herstellungsverfahren nach Anspruch 12, dadurch **gekennzeichnet** , daß es die vorherige Darstellung des Metallocenesters der Formel (V) durch Veresterung der entsprechenden Metallocenylessigsäure, gefolgt von einer Alkylierungsreaktion des Kohlenstoffatoms in der α-Position des Metallocenylcyclus des genannten Esters, umfaßt.

14. Herstellungsverfahren nach Anspruch 12 oder Anspruch 13, dadurch **gekennzeichnet**, daß es zusätzlich einen Schritt zur Trennung der cis- und trans-Isomeren der erhaltenen Verbindung der speziellen Formel (I-a1) umfaßt.

15. Verfahren zur Herstellung der Verbindungen der speziellen Formel (I-a2) nach Anspruch 8 oder Anspruch 9, dadurch **gekennzeichnet**, daß:
a) man ein Chlorid einer Metallocensäure der Formel (VI) : 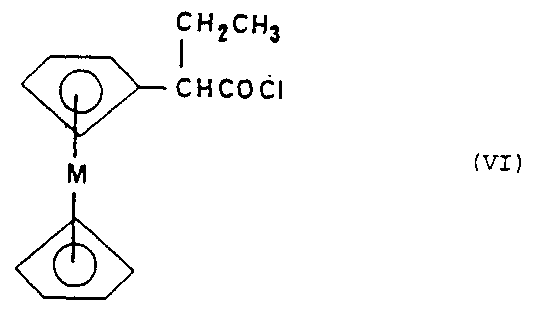 worin M für ein Metallatom, ausgewählt aus Eisen, Ruthenium oder Osmium, steht, mit Diphenylzink umsetzt, um die Substitution des Chloratoms durch eine Phenylgruppe zu erhalten,
b) man die in Schritt a) erhaltene Verbindung einer Additionsreaktion mit der Organolithiumverbindung (CH₃)₂-N-(CH₂)₂-O-C₆H₄-Li oder einer Organomagnesiumverbindung der Formel: (CH₃)₂-N-(CH₂)₂-O-C₆H₄-MgX, in der X für ein Halogenatom, ausgewählt aus Chlor, Brom oder Iod, steht, unterwirft, die zur Bildung eines intermediären Alkohols führt, gefolgt von einer Dehydratisierungsreaktion des genannten Alkohols.

16. Herstellungsverfahren nach Anspruch 15, dadurch **gekennzeichnet** , daß es außerdem einen Schritt zur Trennung der cis- und trans-Isomeren der erhaltenen Verbindung der speziellen Formel (I-a2) umfaßt.

17. Verfahren zur Herstellung einer Verbindung der speziellen Formel (I-b) nach Anspruch 10, dadurch **gekennzeichnet** , daß:
a) man ein Tetralon der Formel (VII): 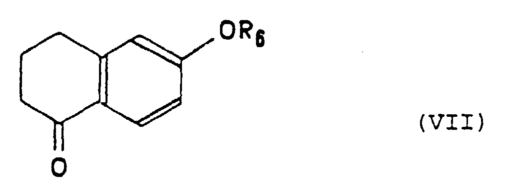 worin R₆ für ein Wasserstoffatom oder eine C₁-C₃-Alkylgruppe steht, mit der Organolithiumverbindung 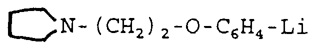 oder einer Organomagnesiumverbindung der Formel: 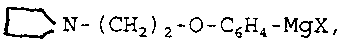 in der X für ein Halogenatom, ausgewählt aus Chlor, Brom oder Iod, steht, umsetzt, was zur Bildung eines intermediären Alkohols führt, gefolgt von einer Dehydratisierungsreaktion des genannten Alkohols,
b) man die in Schritt a) erhaltene Verbindung einer Bromierungsreaktion unterwirft, um die Verbindung der Formel (VIII) zu erhalten: 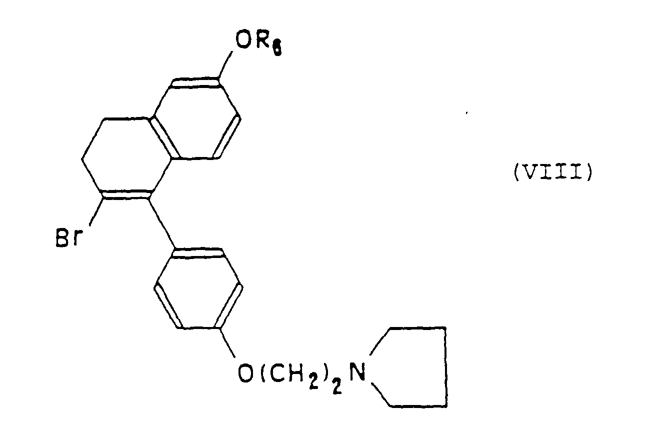
c) man die so erhaltene Verbindung der Formel (VIII) einer Kupplungsreaktion mit einer Organometallverbindung der Formel: Cp-M-(C₅H₄)-Y, in der Cp für eine Cyclopentadienylgruppe steht, M für ein Metallatom, ausgewählt aus Eisen, Ruthenium oder Osmium, steht, und Y für ein Lithiumatom, ein Kupferatom, eine SnBu₃-Gruppe oder eine HgCl-Gruppe steht, unterwirft.

18. Pharmazeutische Zusammensetzung, dadurch **gekennzeichnet**, daß sie mindestens eine der Verbindungen der Formel (I) nach einem der Ansprüche 1 bis 10 oder eines ihrer Salze enthält, gegebenenfalls mit einem pharmazeutisch annehmbaren Träger.

19. Verwendung einer Verbindung der Formel (I) oder eines ihrer Salze nach einem der Ansprüche 1 bis 10 zur Herstellung eines Antitumormedikaments.

## Claims

1. A compound of general formula (I): 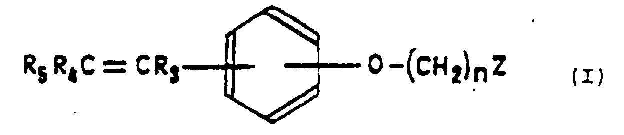 wherein:
the -O(CH₂)ₙZ group is in the meta or para position with respect to the -CR₃=CR₄R₅ group,
n represents an integer of between 2 and 10,
Z represents a basic or polar ligand chosen from -NR₁R₂ groups wherein R₁ and R₂, which are identical or different, represent a C₁ to C₆ alkyl group optionally substituted by one or a number of halogen atoms, heterocyclic groups containing a nitrogen atom or -OR₁, -SR₁, -SOR₁ or -SO₂R₁ groups wherein R₁ has the same meaning as above,
and wherein:
* either R₃ represents a phenyl group optionally substituted by one or a number of hydrogen atoms or halogen atoms or hydroxyl groups or C₁ to C₃ alkyl or alkoxy groups and R₅ represents a metallocene group, whereas R₄ represents a hydrogen atom or a halogen atom or an NO₂ group or a chloroethyl group or a CN group or alternatively a C₁ to C₃ alkyl group, the end carbon of which can optionally be bonded to the carbon situated at the 2 position of the phenyl group of R₃,
* or R₃ represents a metallocene group and R₅ represents a phenyl group optionally substituted by one or a number of hydrogen atoms or halogen atoms or hydroxyl groups or C₁ to C₃ alkyl or alkoxy groups, whereas R₄ represents a hydrogen atom or a halogen atom or an NO₂ group or a chloroethyl group or a CN group or alternatively a C₁ to C₃ alkyl group,
in all its stereoisomeric forms and its salts.

2. The compound as claimed in claim 1, wherein the metallocene group corresponds to the general formula (II) : 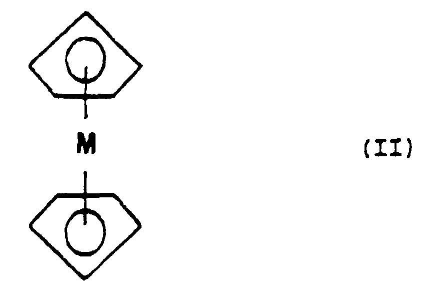 wherein M represents a metal atom from Group VIII of the Periodic Classification of the chemical elements, it being possible for said atom optionally to be in an oxidized form.

3. The compound as claimed in claim 1, wherein the metallocene group corresponds to the general formula (III): 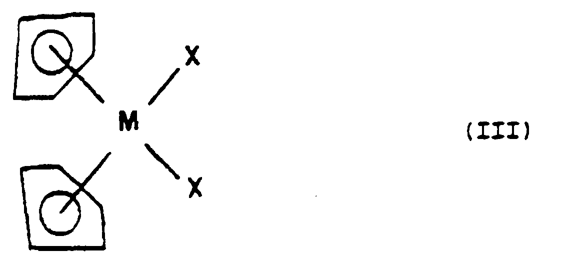 wherein:
M represents a metal atom chosen from titanium, vanadium, niobium, hafnium or molybdenum,
X represents a halogen atom chosen from chlorine, bromine or iodine.

4. The compound as claimed in claim 1 or claim 2, wherein it corresponds to the specific formula (I-a): 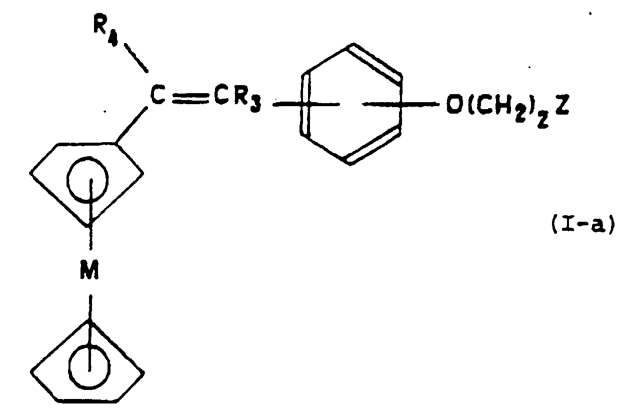 wherein:
the -O(CH₂)₂Z group is in the para position with respect to the -CR₃=CR₄R₅ group,
Z represents an -NR₁R₂ group wherein R₁ and R₂, which are identical or different, represent a methyl group or an ethyl group or alternatively Z represents an N-pyrrolidino group,
R₃ represents a phenyl group optionally substituted by a hydroxyl group or by a methoxy group,
R₄ represents a hydrogen atom or a halogen atom or an NO₂ group or a chloroethyl group or a CN group or alternatively an ethyl group, and
M represents a metal atom chosen from iron, ruthenium or osmium, optionally in its oxidized form.

5. The compound as claimed in claim 4, wherein R₁ and R₂ represent a methyl group, R₃ represents a phenyl group substituted in the para position by a hydroxyl group and R₄ represents an ethyl group, this compound corresponding to the specific formula (I-a1).

6. The compound as claimed in claim 5, wherein it is 1-[4-(2-dimethylaminoethoxy)phenyl]-1-(4-hydroxyphenyl)-2-ferrocenyl-1-butene.

7. The compound as claimed in claim 6, wherein it is the "trans" isomer of 1-[4-(2-dimethylaminoethoxy)phenyl]-1-(4-hydroxyphenyl)-2-ferrocenyl-1-butene.

8. The compound as claimed in claim 4, wherein R₁ and R₂ represent a methyl group, R₃ represents a phenyl group and R₄ represents an ethyl group, this compound corresponding to the specific formula (I-a2).

9. The compound as claimed in claim 8, wherein it is 1-[4-(2-dimethylaminoethoxy)phenyl]-1-phenyl-2-ferrocenyl-1-butene.

10. The compound as claimed in claim 1 or claim 2, wherein:
the -O(CH₂)ₙZ group is in the para position with respect to the -CR₃=CR₄R₅ group,
n is equal to 2 and Z represents an N-pyrrolidino group,
R₃ represents a phenyl group substituted in the para position by a hydroxyl or C₁ to C₃ alkoxy group, whereas R₄ represents a C₂ alkyl group, the end carbon of which is bonded to the carbon situated in the 2 position of the phenyl group of R₃,
M represents a metal atom chosen from iron, ruthenium or osmium, optionally in its oxidized form,
this compound corresponding to the specific formula (I-b).

11. A process for the preparation of the compound as claimed in any one of claims 1 to 10, which comprises:
a) reacting a compound of general formula (IV): 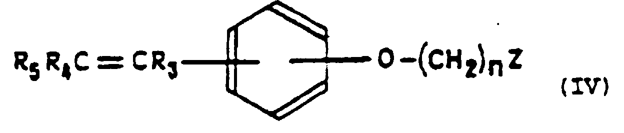 wherein:
the -O(CH₂)ₙZ group is in the meta or para position with respect to the -CR₃=CR₄R₅ group,
n represents an integer of between 2 and 10,
Z represents a basic or polar ligand chosen from -NR₁R₂ groups wherein R₁ and R₂, which are identical or different, represent a C₁ to C₆ alkyl group optionally substituted by one or a number of halogen atoms, heterocyclic groups containing a nitrogen atom or -OR₁, -SR₁, -SOR₁ or -SO₂R₁ groups wherein R₁ has the same meaning as above,
and wherein:
* either R₃ represents a phenyl group optionally substituted by one or a number of hydrogen atoms or halogen atoms or hydroxyl groups or C₁ to C₃ alkyl or alkoxy groups and R₅ represents a bromine atom, whereas R₄ represents a hydrogen atom or a halogen atom or an NO₂ group or a chloroethyl group or a CN group or alternatively a C₁ to C₃ alkyl group, the end carbon of which can optionally be bonded to the carbon situated at the 2 position of the phenyl group of R₃,
* or R₃ represents a bromine atom and R₅ represents a phenyl group optionally substituted by one or a number of hydrogen atoms or halogen atoms or hydroxyl groups or C₁ to C₃ alkyl or alkoxy groups, whereas R₄ represents a hydrogen atom or a halogen atom or an NO₂ group or a chloroethyl group or a CN group or alternatively a C₁ to C₃ alkyl group,
with a cyclopentadienyl metal salt, in order to substitute the bromine atom by a cyclopentadiene group;
b) reacting the compound obtained in Stage a) with a strong base, in order to form a cyclopentadienyl anion,
c) reacting the compound obtained in Stage b) with an organometallic compound of formula:
Cp-M-(CO)₂-X, wherein Cp represents a cyclopentadiene group, M represents a metal atom from Group VIII of the Periodic Classification of the chemical elements and X represents a halogen atom chosen from chlorine, bromine or iodine,
or with an organometallic compound of formula: Cp-M-Xₘ, wherein Cp and X have the same meaning as above, M is a metal atom chosen from titanium, vanadium, niobium, hafnium or molybdenum and m is an integer equal to 3 or 4.

12. A process for the preparation of the compound of specific formula (I-a1) as claimed in any one of claims 5 to 7, which comprises:
a) subjecting a metallocene ester of formula (V): 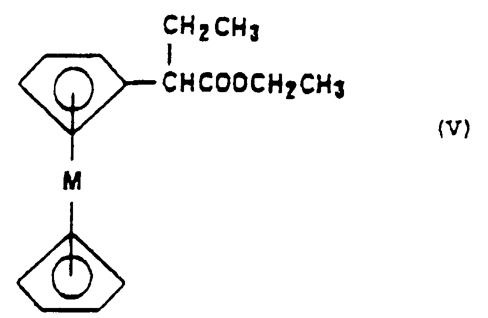 wherein M represents a metal atom chosen from iron, ruthenium or osmium, to an addition reaction with the organolithium compound CH₃-O-C₆H₄-Li or an organomagnesium compound of formula: CH₃-O-C₆H₄-MgX, wherein X represents a chlorine, bromine or iodine atom, which results in the formation of an intermediate alcohol, followed by a dehydration reaction of said alcohol,
b) subjecting the compound obtained in Stage a) to a demethylation reaction,
c) subjecting the compound obtained in Stage b) to an alkylation reaction with sodium ethoxide and the hydrochloride of 2-dimethylaminoethyl chloride.

13. The preparation process as claimed in claim 12, wherein it comprises the prior preparation of the metallocene ester of formula (V) by esterification of the corresponding metallocenylacetic acid, followed by an alkylation reaction of the carbon in the position a to the metallocenyl ring of said ester.

14. The preparation process as claimed in claim 12 or claim 13, wherein it additionally comprises a stage of separation of the "cis" and "trans" isomers of the compound of specific formula (I-a1) obtained.

15. A process for the preparation of the compound of specific formula (I-a2) as claimed in claim 8 or claim 9, which comprises:
a) reacting a metallocenic acid chloride of formula (VI): 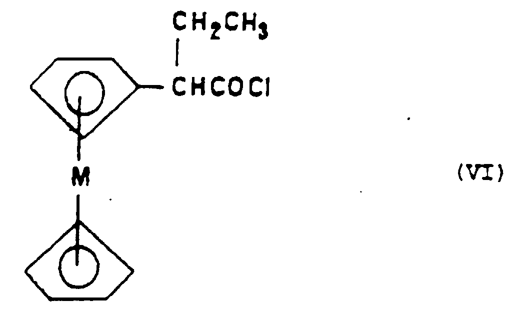 wherein M represents a metal atom chosen from iron, ruthenium or osmium, with diphenylzinc, in order to substitute the chlorine atom by a phenyl group,
b) subjecting the compound obtained in Stage a) to an addition reaction with the organolithium compound (CH₃)₂-N-(CH₂)₂-O-C₆H₄-Li or an organomagnesium compound of formula: (CH₃)₂-N-(CH₂)₂-O-C₆H₄-MgX, wherein X represents a halogen atom chosen from chlorine, bromine or iodine, which results in the formation of an intermediate alcohol, followed by a dehydration reaction of said alcohol.

16. The preparation process as claimed in claim 15, wherein it comprises in addition a stage of separation of the "cis" and "trans" isomers of the compound of specific formula (I-a2) obtained.

17. A process for the preparation of the compound of specific formula (I-b) as claimed in claim 10, which comprises:
a) reacting a tetralone of formula (VII): 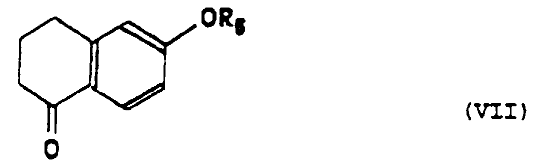 wherein R₆ represents a hydrogen atom or a C₁ to C₃ alkyl group, with the organolithium compound 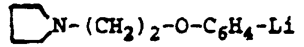 or an organomagnesium compound of formula: 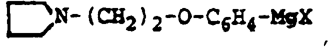 wherein X represents a halogen atom chosen from chlorine, bromine or iodine, which results in the formation of an intermediate alcohol, followed by a dehydration reaction of said alcohol;
b) subjecting the compound obtained in Stage a) to a bromination reaction, in order to obtain the compound of formula (VIII): 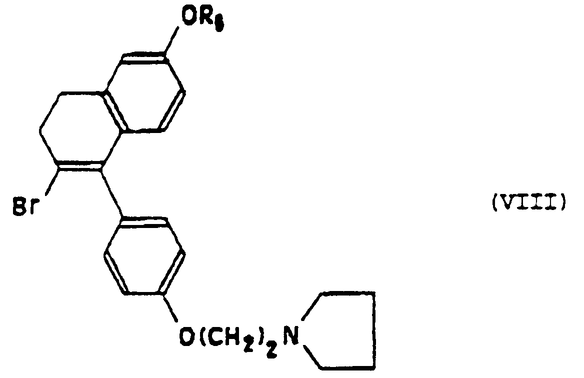
c) subjecting the compound of formula (VIII) thus obtained to a coupling reaction with an organometallic compound of formula: Cp-M-(C₅H₄)-Y, wherein Cp represents a cyclopentadiene group, M represents a metal atom chosen from iron, ruthenium or osmium and Y represents a lithium atom, a copper atom, an SnBu₃ group or an HgCl group.

18. A pharmaceutical composition, wherein it contains at least one of the compounds of formula (I) as claimed in any one of claims 1 to 10 or one of its salts, optionally in combination with a pharmacologically acceptable vehicle.

19. A use of the compound of formula (I) or of one of its salts as claimed in any one of claims 1 to 10 for the production of an antitumor medicament.
